# EUROPEAN PATENT SPECIFICATION

(11) **EP 2 996 701 B1**
(45) Date of publication and mention of the grant of the patent: **24.06.2020**
(21) Application number: 14733289.4
(22) Date of filing: 14.05.2014
(51) Int. Cl.: A61K 35/747, A61P 15/02

(54) **COMPOSITION COMPRISING LACTIC ACID BACTERIA FOR USE IN THE PREVENTIVE AND/OR CURATIVE TREATMENT OF BACTERIAL VAGINOSIS**
ZUSAMMENSETZUNG UMFASSEND MILCHSÄUREBAKTERIE ZUR VERWENDUNG ZUR VORBEUGENDEN UND/ODER KURATIVEN BEHANDLUNG VON BAKTERIELLE VAGINOSE
COMPOSITION CONTENANT DES BACTERIES D'ACIDE LACTIQUE POUR SON UTILISATION DANS LE TRAITEMENT PREVENTIF ET/OU CURATIF DE LA VAGINOSE BACTÉRIENNE

(30) Priority: 14.05.2013 IT MI20130794
(43) Date of publication of application: 23.03.2016
(73) Proprietor: Probiotical S.p.A., 28100 Novara (IT)
(72) Inventor: MOGNA, Giovanni, I-28100 Novara NO (IT)
(74) Representative: Hoffmann Eitle
(86) International application number: PCT/IB2014/000739
(87) International publication number: WO 2014/184643

(56) References cited:
- EP-A1- 2 000 530
- EP-A1- 2 269 465
- WO-A1-2006/013588
- WO-A1-2010/099824
- WO-A1-2010/133761
- WO-A1-2010/136891
- WO-A1-2012/101500
- WO-A1-2013/034974
- WO-A1-2013/034975
- WO-A2-2008/038075
- US-A1- 2010 092 440
- SAGGIORO ALFREDO: "Probiotics in the treatment of irritable bowel syndrome.", JOURNAL OF CLINICAL GASTROENTEROLOGY JUL 2004, vol. 38, no. 6 Suppl, July 2004 (2004-07), pages S104-S106, XP009172163, ISSN: 0192-0790
- MOGNA LUCA ET AL: "Assessment of the in vitro inhibitory activity of specific probiotic bacteria against different Escherichia coli strains", JOURNAL OF CLINICAL GASTROENTEROLOGY UNITED STATES, USA, vol. 46, no. Suppl, 1 October 2012 (2012-10-01), pages S29-S32, XP009167929, ISSN: 1539-2031, DOI: 10.1097/MCG.0B013E31826852B7
- VIOLETA CASTRO-LEYVA ET AL: "Preserved Ex Vivo Inflammatory Status in Decidual Cells from Women with Preterm Labor and Subclinical Intrauterine Infection", PLOS ONE, vol. 7, no. 8, 22 August 2012 (2012-08-22) , page e43605, XP055077158, DOI: 10.1371/journal.pone.0043605
- ISMAIL AL-WAHSH ET AL: "Acute probiotic ingestion reduces gastrointestinal oxalate absorption in healthy subjects", UROLOGICAL RESEARCH ; A JOURNAL OF CLINICAL AND LABORATORY INVESTIGATION IN UROLITHIASIS AND RELATED AREAS, SPRINGER, BERLIN, DE, vol. 40, no. 3, 28 August 2011 (2011-08-28), pages 191-196, XP035058023, ISSN: 1434-0879, DOI: 10.1007/S00240-011-0421-7
- HOESL C E ET AL: "The Probiotic Approach: An Alternative Treatment Option in Urology", EUROPEAN UROLOGY, ELSEVIER BV, NL, vol. 47, no. 3, 1 March 2005 (2005-03-01), pages 288-296, XP027606331, ISSN: 0302-2838 [retrieved on 2005-03-01]
- STRUS M ET AL: "Studies on the effects of probiotic Lactobacillus mixture given orally on vaginal and rectal colonization and on parameters of vaginal health in women with intermediate vaginal flora", EUROPEAN JOURNAL OF OBSTETRICS GYNECOLOGY AND REPRODUCTIVE BIOLOGY 2012 ELSEVIER IRELAND LTD IRL, vol. 163, no. 2, August 2012 (2012-08), pages 210-215, XP002712127, ISSN: 0301-2115
- L. PASCUAL ET AL: "Vaginal colonization and activity of the probiotic bacterium Lactobacillus fermentum L23 in a murine model of vaginal tract infection", JOURNAL OF MEDICAL MICROBIOLOGY, vol. 59, no. 3, 19 November 2009 (2009-11-19), pages 360-364, XP055077489, ISSN: 0022-2615, DOI: 10.1099/jmm.0.012583-0
- "7th Probiotics & Prebiotics - new foods", , 30 July 2013 (2013-07-30), pages Cover-50, XP055130413, Rome, Università Urbaniana Retrieved from the Internet: URL:http://www.probiotics-prebiotics-newfo od.com/media/scientific-programme.pdf [retrieved on 2014-07-21]

## Description

The present invention relates to a composition comprising lactic bacteria for topical vaginal use. The composition of the present invention is effectively applicable in the preventive and/or curative treatment of vaginal infections caused by vaginal pathogens, with particular reference to *Gardnerella vaginalis* and/or coliform bacteria, responsible for bacterial vaginosis, even recurrent or relapsing.

It is known that the vaginal microbiota consists of a pool of endogenous microorganisms with various size and nature, which physiologically live as commensal colonizing the eco-environment, and which thus, by definition, form the ecosystem thereof. Such an ecosystem is represented, on the whole, by the epithelial cells lining the vagina and uterus, by the glandular ones secreting in the lumen of the organ and by the complex bacterial flora, which symbiotically interacts for maintaining a proper physiological status. The equilibrium condition thus obtained supports a dynamics which alternates with physiopathological phenomena, wherein the environment influences the microbiota, and the microorganisms, in turn, through a mainly ascending propagation, influence the reactivity of the cell-mediated immune defence. The vaginal microbial population is mixed and comprises about 50 bacterial species, both aerobic and anaerobic. Usually the 95% of the microbial component is represented by lactobacilli, which form part of the "Doderlein complex", whereas the remaining 5% comprises commensal bacteria and opportunistic pathogens, among which *Streptococcus epidermidis,* Diphtheroids, Peptostreptococci, *Bacteroides* spp., *Escherichia coli, Gardnerella vaginalis* and Candida spp.

Therefore, the strains belonging to the genus *Lactobacillus* represent the prevalent component, as shown by both standard analytical and molecular techniques: the concentration of such microorganisms is of about 1x10⁹ CFU/ml, a value about 100-fold greater than the concentration of the same bacterial group per gram of intestinal content. The most represented species of lactobacilli are *L. acidophilus, L. fermentum, L. plantarum, L. brevis, L. jensenii, L. casei, L. cellobiosus, L. leichmanii, L. delbrueckii and L. salivarius.*

Several studies concerning the vaginal microbiota indicate that lactobacilli, usually predominantly present, play a key role in preventing colonization by unwanted microorganisms, among which those responsible for bacterial vaginosis, fungal infections, sexually transmitted diseases and urinary tract infections (UTI).

The vaginal wall cells have a main role in maintaining such an equilibrium, in particular the upper and middle layers of epithelium, the proliferation and maturation of which is hormone-related. The estrogen levels, in fact, affect the glycogen content of the cells of the vaginal epithelium and the metabolism of the latter mediates the synthesis of organic acids, reducing the pH of the lumen of the organ up to values comprised between 3.5 and 4.5. Due to this mechanism, the invasion of exogenous pathogens or the replication of harmless microorganisms, usually existing at vaginal level at low amounts, is hindered. In prepubescent subjects the glycogen content of the vaginal epithelium is low and the pH is about 7.

However, it is quite frequent that, due to different exogenous and endogenous factors, such as antibiotic intake, stress conditions, hormonal modulations related to pregnancy, menstrual cycle or the intake of high estrogen concentrations, an unbalance of this complex and equilibrated ecosystem occurs.

Therefore, there is a need for effectively intervene in order to re-equilibrate and balance the vaginal ecosystem for avoiding that the alteration of the microbiota equilibrium leads to a prevalence of opportunistic microorganisms which, no longer hindered by the action of the "Doderlein complex", can cause vaginitis or vaginosis forms caused by opportunistic germs, among which *Gardnerella* and/or coliforms.

In postmenopausal women, in which the drop of estrogen levels leads to a reduction of the vaginal glycogen concentration, the vaginal pH increases and the anaerobic bacteria become dominant so that a significant proliferation of *Enterobacteriaceae* takes place.

Bacterial vaginosis is the most common vaginal infection among women during reproductive age and it is also frequent among pregnant women. Bacterial vaginosis represents more than 60% of the overall vaginal infections. The *National Center for Health Statistics* - *National Health and Nutrition Survey (NHANES)* in a survey carried out on more than 12.000 women in USA on 2001-2004 showed that the prevalence of bacterial vaginosis was 29.2%, but only 15.7% was symptomatic. A good percentage of vaginosis is thus asymptomatic, namely does not lead to symptoms typical of vaginal inflammations such as itching, burning and pain during sexual activity. Many of them, inter alia, do not either cause irritations, redness or swelling but, in any case, they cause an increase of vaginal discharges, with white or grayish secretions, characterized by a bad smell. Due to the fact that vaginal discharges substantially change depending on the menstrual cycle stage, the woman is often unable to understand that there is an ongoing infection and vaginosis is not diagnosed. The non-treatment of a vaginosis can lead to the onset of even severe vaginitis and infections, mainly in particularly sensitive subjects. Furthermore, it can contribute in transmitting infections through the sexual activity.

Therefore, there is a need to effectively intervene for preventing and/or curing the onset of vaginosis and, consequently, of vaginitis and bacterial infections.

The real cause of bacterial vaginosis is still presently an object of study and investigation. The main agent responsible for most of the cases is the Gram-variable bacterium *Gardnerella vaginalis* (80-85%) the only known species of genus Gardnerella, and the Gram-negative bacterium E.coli (10-15%). Additionally, the pathogenic bacterium *E.coli* is also responsible for aerobic vaginosis, which causes the risk of preterm births.

Nevertheless, a bacterial vaginosis onset is related to the simultaneous interaction with other risk factors. Among the most significant risk factors, for example, an improper personal hygiene (for example an undue use of vaginal douches), the use of antibiotics or intrauterine mechanical contraceptives such as the coil, previous pregnancies and some genetic predisposition have to be cited.

Women with bacterial vaginosis have abnormal vaginal discharges, with an unpleasant smell. The possible discharges are usually of white or grey color and can be quite fluid. Furthermore, affected women experience burning during urination and/or external genital itching. However, as already explained, a fair part of affected women do not have any symptom.

In most of the cases, bacterial vaginosis does not cause any severe problem. Nevertheless, some quite severe potential complications have to be taken into account such as, for example, the increased risk of HIV infection, in the event of exposure to the virus, or the risk of infection by etiological agents of other sexually transmitted diseases, among which the genital virus Herpes simplex, Chlamydia and gonorrhea.

Bacteria causing vaginosis can also infect the uterus and Fallopian tubes. Such a kind of infection is named pelvic inflammatory disease and can lead to infertility or impair the tubes, increasing the risk of ectopic pregnancies and infertility.

It is known that bacterial vaginosis can be treated with molecules with antibiotic activity, among which metronidazole or clindamycin are very common. Both can be used even during pregnancy, but the recommended dosage is different. Although these actions are generally considered effective in the treatment of acute infections, they are often unable to provide a significant protection against possible relapses, which may occur at various times after the end of the therapy. Furthermore, the antibiotic molecules presently available still represent an approach based on organic synthetic drugs and, as such, clearly extraneous to the physiological habitat of the vagina.

Thus, there is a need to effectively intervene for preventing and/or curing the onset of vaginosis and, consequently, of vaginitis and bacterial infections avoiding the use of synthetic molecules but, on the contrary, by adopting a treatment compatible with the physiological habitat of the vagina.

Moreover, there is a need to have a natural and effective barrier effect, in the case of both an acute episode and possible relapses, which continues over the time in order to ensure a long-term protection and assure a long-lasting effective preventive and/or curative treatment.

The Applicant, following to an extended and intense research activity, gave a response to the above-cited needs. In fact, the Applicant, after testing and studying many lactic bacteria, succeeded to select only some of them based on their specific activity in colonizing the vaginal mucosa and producing bacteriostatic and bactericidal molecules. The selected strains are effective against *Gardnerella vaginalis* and are anti-*E.coli* also performing at the same time an anti-inflammatory activity since they are strains producing interleukin 10 (IL-10) and interleukin 4 (IL-4). The selected strains having these specific proprieties (anti-E.coli and anti-inflammatory) are effective against bacterial vaginosis, vaginitis and related infections.

It is an object of the present invention to provide a composition for use in the preventive and/or curative treatment of bacterial vaginosis, preferably of recurrent or relapsing vaginosis, having the characteristics as described in the appended claim.

Preferred embodiments of the present invention are contemplated in the following detailed description.

In the context of the present invention by composition is meant a pharmaceutical composition, or a supplement product or a medical device or a food composition.

It is an object of the present invention a strain of bacteria belonging to the species *Lactobacillus fermentum* deposited by Probiotical S.p.A. Company on 03.01.2013 at the DSMZ center and having deposit number DSM 26955 (LF15) and a strain of bacteria belonging to the species *Lactobacillus fermentum* deposited by Probiotical S.p.A. Company on 03.01.2013 at the DSMZ center and having deposit number DSM 26956 (LF16), for use in the preventive and/or curative treatment of bacterial vaginosis, vaginitis and vaginal infections related with coliform pathogenic bacteria and/or Gardnerella.

It is an object of the present invention a composition comprising a mixture of bacteria, which comprises or, alternatively, consists of strains of bacteria having both a specific antibacterial activity against *Gardnerella vaginalis* and *E.coli* and an anti-inflammatory activity with stimulation of the Interleukin IL-4 and Interleukin IL-10 production.

It is an object of the present invention a composition wherein said mixture comprises or, alternatively, consists of at least a strain of bacteria having a specific antibacterial activity against *Gardnerella vaginalis* combined with at least a strain having an antibacterial *E.coli* activity and an anti-inflammatory activity with stimulation of the Interleukin IL-4 and Interleukin IL-10 production.

It is an object of the present invention a composition comprising or, alternatively, consisting of a mixture of bacteria which comprises or, alternatively, consists of at least a strain of bacteria selected from the group comprising the strain *Lactobacillus plantarum* LMG P-21021 -LP01 and the strain *Lactobacillus plantarum* LMG P-21020 -LP02, combined with at least a strain of bacteria selected from the group comprising the strain of bacteria *Lactobacillus fermentum* DSM 26955 (LF15) and the strain of bacteria *Lactobacillus fermentum* DSM 26956 (LF16), for use in the preventive and/or curative treatment of bacterial vaginosis, vaginitis and vaginal infections related with coliform pathogens and/or Gardnerella; preferably for the treatment of recurrent or relapsing vaginosis.

The strain *Lactobacillus plantarum* LMG P-21021 -LP01 was deposited by Mofin Srl Company on 16.10.2001 at the BCCM LMG center.

The strain *Lactobacillus plantarum* LMG P-21020 -LP02 was deposited by Mofin Srl Company on 16.10.2001 at the BCCM LMG center.

In a preferred embodiment, the composition of the present invention comprising or, alternatively, consisting of a mixture of bacteria which comprises or, alternatively, consists of the strain of bacteria *Lactobacillus plantarum* LMG P-21021 -LP01, combined with at least a strain of bacteria selected from the group comprising the strain of bacteria *Lactobacillus fermentum* DSM 26955 (LF15) and the strain of bacteria *Lactobacillus fermentum* DSM 26956 (LF16), for use in the preventive and/or curative treatment of bacterial vaginosis, vaginitis and vaginal infections related with coliform pathogens and/or Gardnerella; preferably for the treatment of recurrent or relapsing vaginosis.

Preferably, said composition comprising or, alternatively, consisting of a mixture of bacteria which comprises or, alternatively, consists of the strain of bacteria *Lactobacillus plantarum* LMG P-21021 -LP01 and the strain of bacteria *Lactobacillus fermentum* DSM 26955 (LF15).

In another preferred embodiment, the composition of the present invention comprising or, alternatively, consisting of a mixture of bacteria which comprises or, alternatively, consists of the strain of bacteria *Lactobacillus plantarum* LMG P-21020 -LP02, combined with at least a strain of bacteria selected from the group comprising the strain of bacteria *Lactobacillus fermentum* DSM 26955 (LF15) and the strain of bacteria *Lactobacillus fermentum* DSM 26956 (LF16), for use in the preventive and/or curative treatment of bacterial vaginosis, vaginitis and vaginal infections related with coliform pathogens and/or Gardnerella; preferably for the treatment of recurrent or relapsing vaginosis.

Preferably, said composition comprising or, alternatively, consisting of a mixture of bacteria which comprises or, alternatively, consists of the strain of bacteria *Lactobacillus plantarum* LMG P-21020 -LP02 and the strain of bacteria *Lactobacillus fermentum* DSM 26956 (LF16).

In another embodiment, the composition of the present invention further comprises a strain of bacteria selected from the group comprising or, alternatively, consisting of a strain *Lactobacillus fermentum* DSM 18296 (LF07) deposited on 24.05.2006 by Probiotical S.p.A Company.

The composition of the present invention further comprises at least a gelling substance, preferably having a marked muco-adhesive property, such as a natural gum or a plant gum and/or a plant gelatin. The plant gum and/or the plant gelatin is preferably selected from the group comprising a tannate or a gelatin tannate, an alginate, a xyloglucan or xylogel, a guar gum, a tara gum, an acacia, carob, oat, bamboo fiber, citrus fruit fibers and glucomannans. Preferably said gelling substance is selected from galactomannans of natural origin. In a preferred embodiment, the gelling substance is selected from guar gums.

Additionally, the composition of the present invention further comprises at least a fiber with prebiotic activity selected from the group comprising inulin, fructo-oligosaccharides (FOS), galacto- and trans-galacto-oligosaccharides (GOS and TOS), gluco-oligosaccharides (GOSα), xylo-oligosaccharides, (XOS), chitosan-oligosaccharides (COS), soy-oligosaccharides (SOS), isomalto-oligosaccharides (IMOS), resistant starch, pectins, psyllium, arabino-galactans, gluco-mannans and galacto-mannans. In an embodiment, said fiber with prebiotic activity is selected from fructo-oligosaccharides (FOS), arabinogalactans or mixtures thereof. An embodiment of the present invention is represented by a composition, which comprises or, alternatively, consists of a strain of bacteria *Lactobacillus fermentum* DSM 26955 (LF15), a strain of bacteria *Lactobacillus plantarum* LMG P-21021 (LP01) and/or a guar gum and/or a prebiotic fiber selected from arabinogalactan and fructo-oligosaccharide (FOS) or mixtures thereof.

Another embodiment of the present invention is represented by a composition, which comprises or, alternatively, consists of a strain of bacteria *Lactobacillus fermentum* DSM 26956 (LF16), a strain of bacteria *Lactobacillus plantarum* LMG P-21020 (LP02) and/or a guar gum and/or a prebiotic fiber selected from arabinogalactan and fructo-oligosaccharide (FOS) or mixtures thereof.

The composition of the present invention may be in solid form, for topical vaginal use, as a tablet, a capsule, an ovule, a lozenge or granules or powder; or in liquid form, for topical vaginal use, as a solution, a liquid vaginal douche, a suspension or a gel.

The composition of the present invention contains excipients and technological additives, known to the skilled in the field, suitable for preparing a solid form for internal use, which is able to disintegrate and release the strains of bacteria after the hydration of said solid form inside the vaginal cavity.

In addition, the composition of the present invention is prepared by techniques and equipment known to the skilled in the field which is able to suitably adopt the compressibility, temperature and concentration parameters of the various excipients being used in order to avoid that the bacterial load contained in a solid form, for example a tablet or an ovule for internal use, is dramatically reduced over the time and/or that the bacterial cell suffers from the preparation process to such an extent to compromise its health condition and viability.

The composition of the present invention in the form of tablet or ovule has an optimum survival of the two lactobacilli both during the compression step and during the commercial life of the product at a storage temperature not greater than 25°C. The composition of the present invention in solid form for topical vaginal use has a shelf-life of 2 years at room temperature ensuring a minimum bacterial load of at least 400x10⁶ viable cells, for each strain of bacterium therein contained, per tablet or ovule.

The presence of the gelling agent guar gum, together with the strains of bacteria of the present invention *L*. *fermentum* LF15 and *L*. *plantarum* LP01 or *L*. *fermentum* LF16 and *L. plantarum* LP02, is able to rapidly establish an effective barrier effect against potential vaginal pathogens, with particular reference to *Gardnerella vaginalis* and/or coliform bacteria, well known causes of vaginosis, even relapsing. The efficacy is against bacterial vaginosis, mainly when is caused by *Gardnerella vaginalis* and/or coliform bacteria.

The selected strains *L. fermentum* LF15 and *L*. *fermentum* LF16 are effective against *Gardnerella vaginalis,* whereas the selected strains *L*. *plantarum* LP01 and *L. plantarum* LP02 are active against *E.coli* and exert at the same time an anti-inflammatory activity since they are strains producing interleukin 10 (IL-10) and interleukin 4 (IL-4).

The use of the muco-adhesive gelling agent, selected from those described above, such as for example guar gum (natural galactomannan), is able to rapidly establish, through the formation of a hydrogel directly in the vagina and after very few minutes from disaggregation of the tablet or ovule, a mechanical barrier effect against potential vaginal pathogens, with particular reference to *Gardnerella vaginalis* and coliform bacteria, among which *Escherichia coli* has a particular importance.

As soon as the muco-adhesive gelling agent, for example guar gum, hydrates in the vaginal environment, this mediates the formation of a gel having an optimum viscosity which, by evenly distributing over the mucosa, creates a physical-mechanical barrier action against the adhesion of the above-cited commensal or pathogenic bacteria.

The main component of guar gum is a galactomannan, a trisaccharide consisting of mannose and galactose units, specifically polymerized to form α-D-mannopyranosil chains linked by a β-D-(1-4) glycosidic bond and with molecular weight around 200-300 kDa, to form a straight 1-4 chain with short side 1-6 branches of galactose.

The composition of the present invention, in addition to the gelling agent for example the guar gum, contains the two specific microorganisms *Lactobacillus fermentum* LF15 *and Lactobacillus plantarum* LP01 or *Lactobacillus fermentum* LF16 *and Lactobacillus plantarum* LP02, at a concentration comprised from 1x10⁷ to 1x10¹⁰, preferably from 1x10⁸ to 1x10⁹ CFU/g (and in any case not lesser than 400x10⁶ of viable cells per strain, per tablet or ovule), which rapidly integrate in the vaginal microbiota restoring the "Doderlein" complex and, consequently, enhancing the barrier effect.

The composition of the present invention contains the two specific microorganisms *Lactobacillus fermentum* LF15 *and Lactobacillus plantarum* LP01 or *Lactobacillus fermentum* LF16 and *Lactobacillus plantarum* LP02, in a ratio comprised from 3:1 to 1:3, preferably from 2:1 to 2:1, even more preferably 1:1; the gelling substance in an amount comprised from 1 to 50% by weight, preferably from 5 to 35%, even more preferably from 10 to 20%; the prebiotic fiber in an amount comprised from 1 to 50% by weight, preferably from 5 to 35%, even more preferably from 10 to 20%.

In fact, the bacteria of the present invention are able to establish a long-term effective barrier effect due to the colonization of the vaginal mucosa, which leads to a self-regulated production of organic acids, mainly lactic and acetic. These bacteria enhance the barrier effect against commensal or pathogenic bacteria responsible for vaginosis through the synthesis and release, by the two lactobacilli, of molecules with specific activity, mainly bacteriolysins and bacteriocins, in addition to the organic acids (lactic and acetic).

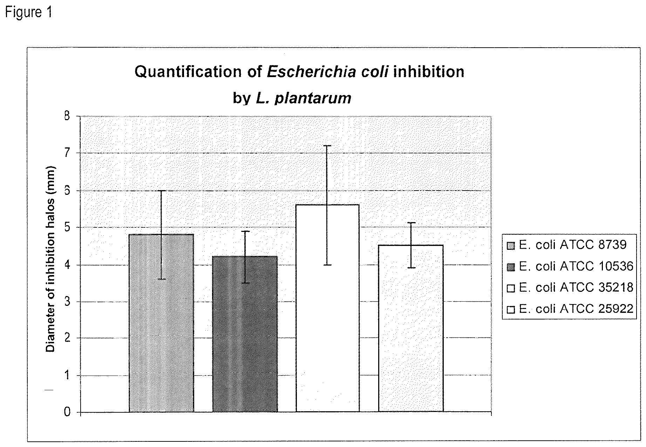

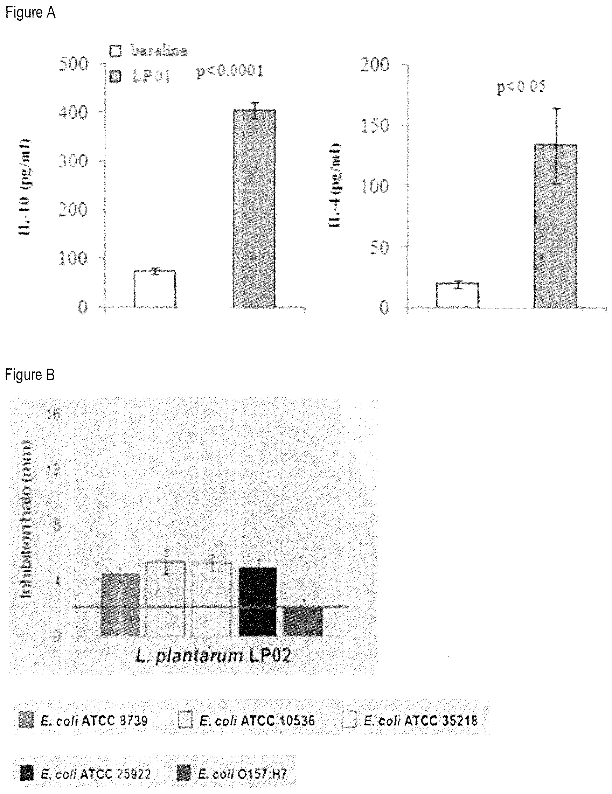

The antagonistic activity of the strain *L. plantarum* LP01 and the strain *L*. *plantarum* LP02 was tested *in vitro* against 4 strains of *Escherichia coli* (ATCC 8739, ATCC 10536, ATCC 35218 and ATCC 25922). Only the results relative to the strain *L*. *plantarum* LP01 (Figure 1) are presented, since both led to similar results.

The results are disclosed in Figure 1. The results are expressed as diameter of inhibition halo (mm) mediated by the lactobacillus on each strain of *Escherichia coli.* The significance threshold of the results was taken equal to 2 mm. An inhibition halo is an area of the dish wherein *E*. *coli* was unable to grow due to the barrier effect exerted by *L. plantarum* LP01. The results are disclosed as means ± standard deviation (SD) of 3 independent experiments.

As it can be noted from histograms (Figure 1), the strain *L. plantarum* LP01 is able to significantly hinder all of the 4 strains of *Escherichia coli* being tested, with inhibition diameters greater than 4 mm.

On the other hand, the strains of bacteria *L. fermentum* LF15 and *L. fermentum* LF16 are able to significantly hinder the rod-shaped, Gram-variable bacterium *Gardnerella vaginalis.* In particular, the antagonistic activity of said lactobacilli was tested against a strain of Gardnerella, by quantification of the optical density detected at the wavelength of 600 nm (OD₆₀₀) after 24 and 48 hours of microaerophilic growth. Gardnerella was grown in a liquid TH medium, whereas each lactobacillus tested in the experiment was cultured in a liquid MRS medium. At the end of the growing, the neutralized supernatants from the cultures of each lactobacillus were added at different percentages to fresh MRS inoculated with *Gardnerella vaginalis* 2%. The growing of Gardnerella alone in fresh MRS (positive control) and with neutralized supernatant amounts comprised between 0.5% and 4%, after 24 and 48 hours. Only the results relative to the strain *L*. *fermentum* LF15 (Table 1 at 24 hours and Table 2 at 48 hours) are shown, since similar results were also obtained for the strain *L. fermentum* LF16.

**Table 1**

| **24 hours** | ***Fresh MRS amount*** | | | |
|---|---|---|---|---|
| | 0.5 | 1 | 2 | 4 |
| *Gardnerella vaginalis* | **1.251** | **1.534** | **1.811** | **1.807** |
| | | | | |

| Neutralized supernatant | ***Supernatant amount*** | | | |
|---|---|---|---|---|
| | 0.5 | 1 | 2 | 4 |
| *L. crispatus* DSM 24439 (ID 1626) | **0.505** | **0.511** | **0.673** | **1.035** |
| *L. crispatus* DSM 24438 (ID 1605) | **0.531** | **0.484** | **0.462** | **0.511** |
| *L. paracasei* DSM 21718 LPC08 (ID 1696) | **0.653** | **1.130** | **1.538** | **1.724** |
| *L. paracasei* DSM 24440 (ID 1608) | **0.689** | **1.214** | **1.476** | **1.774** |
| *L. fermentum* DSM 18289 (ID 1456) | **0.727** | **0.541** | **0.591** | **0.745** |
| *L. fermentum* DSM 18296 (ID 1459) | **0.677** | **0.530** | **0.589** | **0.665** |
| *L. fermentum* DSM 18297 (ID 1460) | **0.681** | **0.583** | **0.717** | **0.710** |
| *L. fermentum* DSM 26955 (LF15) | **0.255** | **0.339** | **0.302** | **0.321** |
| *L. fermentum* DSM 26956 (LF16) | **0.483** | **0.425** | **0.486** | **0.416** |

**Table 2**

| **48 hours** | ***Fresh MRS amount*** | | | |
|---|---|---|---|---|
| | 0.5 | 1 | 2 | 4 |
| *Gardnerella vaginalis* | **0.874** | **1.255** | **1.874** | **1.837** |
| | | | | |

| Neutralized supernatant | *Supernatant amount* | | | |
|---|---|---|---|---|
| | 0.5 | 1 | 2 | 4 |
| *L. crispatus* DSM 24439 (ID 1626) | **0.437** | **0.465** | **0.508** | **0.509** |
| *L. crispatus* DSM 24438 (ID 1605) | **0.367** | **0.369** | **0.362** | **0.380** |
| *L*. *paracasei* DSM 21718 LPC08 (ID 1696) | **0.489** | **0.625** | **1.082** | **1.734** |
| *L. paracasei* DSM 24440 (ID 1608) | **0.522** | **0.591** | **0.879** | **1.748** |
| *L. fermentum* DSM 18289 (ID 1456) | **0.617** | **0.463** | **0.435** | **0.446** |
| *L. fermentum* DSM 18296 (ID 1459) | **0.489** | **0.474** | **0.379** | **0.370** |
| *L. fermentum* DSM 18297 (ID 1460) | **0.510** | **0.532** | **0.472** | **0.438** |
| *L. fermentum* DSM 26955 (LF15) | **0.255** | **0.317** | **0.233** | **0.212** |
| *L. fermentum* DSM 26956 (LF16) | **0.366** | **0.348** | **0.312** | **0.287** |

All the strains of bacterium listed in Table 1 and Table 2 were deposited by the Applicant and are available to the public in accordance with the requirements established by the Budapest Treaty. As expected, the growth of G. *vaginalis* alone in fresh MRS medium is directly proportional to the percentage of medium itself made available to the bacterium. When increasing the percentage of neutralized supernatant, namely brought to neutral pH in order to eliminate the non-specific inhibitory effects mediated by the organic acids produced by lactobacilli, the growth of G. *vaginalis* resulted decreased mainly in the case of *L*. *fermentum* LF15, capable to mediate a totally specific inhibitory action. On the other hand, with *L. paracasei* LPC08 and *L. paracasei* ID 1608 the growing of G. *vaginalis* had values substantially similar to those obtained in fresh MRS, suggesting that these two lactobacilli exert no inhibitory action. The remaining five strains mediate an antagonistic action, mainly *L*. *crispatus* ID 1605 and *L. fermentum* ID 1459, although of a lesser extent than *L. fermentum* LF15. The same applies for the strain *L*. *fermentum* LF16.

In addition to the above-described specific inhibitory mechanisms, both lactobacilli are able to produce high amounts of lactic and acetic acids due to their facultative heterofermentative metabolism, contributing to rapidly and considerably lower the intravaginal pH.

Furthermore, the presence of the two fibers, such as for example arabinogalactan and short chain fructo-oligosaccharides (FOSsc) provides a selective nourishment for the microorganisms of the "Doderlein complex" and lactobacilli existing in the formulation, thereby promoting the ability of colonizing the vaginal ecosystem and the subsequent development.

The competitive advantage deriving from the capability of fermenting these particular carbon sources results in a fast increase of the *L. fermentum* LF15, *L. plantarum* LP01, *L*. *fermentum* LF16 and *L*. *plantarum* LP02 and other autochthonous lactobacilli populations, with subsequent synthesis of remarkable amounts of peptides with antimicrobial action and organic acids able to lowering the vaginal pH to values incompatible with most of the pathogenic species.

Advantageously, the composition of the present invention for topical vaginal use consists of:
- a high bacterial population, for a fast colonization of the overall ecosystem,
- strains of bacteria in a good physiological status, for a prompt multiplication,
- a proper stability, under the storage conditions of the final product, so that to ensure the efficacy until the end of the shelf-life.

The composition of the present invention is effectively applicable in the treatment of recurrent or relapsing vaginosis.

### Cytokines with immunoregulatory action

This study assessed the induction of cytokines IL-4 and IL-10, which represent the main cytokines with immunoregulatory action. As it is shown in figure A, the tested strains of bacteria *Lactobacillus plantarum* LMG P-21021 -LP01 and *Lactobacillus plantarum* LMG P-21020 -LP02 are able to induce a statistically significant growth relative to the basal conditions in both the cytokines.

Figure A: Cytokine profile. Mean ± S.E.M. of 10 independent experiments. The statistical meaning is calculated by using the Student's *t*-test. When calculated relative to the basal conditions (non-stimulated PBMC), the values p<0.05 are considered statistically significant. The IL-10 production was assessed in the culture supernatant after one day from the stimulation. The IL-4 production was assessed in the culture supernatant after five days of stimulation. Similar results were obtained with the strain *Lactobacillus plantarum* LMG P-21020 -LP02.

Figure 1 relates to the quantification of *E.coli* (ATCC 8739, ATCC 10536, ATCC 35218, ATCC 25922) inhibition by the strain *Lactobacillus plantarum* LMG P-21021 -LP01.
Figure B relates to the quantification of *E.coli* (ATCC 8739, ATCC 10536, ATCC 35218, ATCC 25922) inhibition by the strain *Lactobacillus plantarum* LMG P-21020 -LP02.

It is an object of the present invention a composition comprising or, alternatively, consisting of:
- a strain of bacterium *Lactobacillus plantarum* LMG P-21021 (LP01), and/or
- a strain of bacterium *Lactobacillus fermentum* DSM 26955 (LF15), and
- a tara gum, and
- an arabinogalactan fiber, and
- a fructo-oligosaccharide fiber, for use in the preventive and/or curative treatment of bacterial vaginosis, vaginitis and vaginal infections related with coliform pathogens and/or Gardnerella; and for use against recurrent or relapsing vaginosis.

The strain of bacterium *Lactobacillus plantarum* LMG P-21021 (LP01) and the strain of bacterium *Lactobacillus fermentum* DSM 26955 (LF15) are each present at a concentration comprised from 1x10⁷ to 1x10¹⁰, preferably from 1x10⁸ to 1x10⁹ CFU/g (and in any case not lesser than 400x10⁶ of viable cells per strain, per tablet or ovule). The two strains of bacteria of *Lactobacillus* exist in the composition in a weight ratio comprised from 1:3 to 3:1, preferably from 1:2 to 2:1, for example 1:1.

The tara gum is in an amount comprised from 1 to 10% by weight, relative to the weight of the composition, preferably from 3 to 8%, for example 5%.

The arabinogalactan fiber is in an amount comprised from 10 to 45% by weight, relative to the weight of the composition, preferably from 25 to 40%, for example 35%.

The fructo-oligosaccharide fiber is in an amount comprised from 10 to 40% by weight, relative to the weight of the composition, preferably from 15 to 35%, for example 25%.

The weight ratio between arabinogalactan fiber and fructo-oligosaccharide fiber is comprised from 1:2 to 2:1, preferably from 1:1.5 to 1.5:1.

For example, one gram of composition may contain the two strains of bacteria of *Lactobacillus* at a concentration of 4x10⁶ CFU/g each (weight 370 mg), 50 mg of tara gum, arabinogalactan 340 mg and fructo-oligosaccharide 240 mg; said composition can be in the form of slow-release vaginal tablet (composition).

The Applicant tested both the two strains of bacterium *Lactobacillus in vitro* and the composition *in vivo* (study).
i) *In vitro* said strains of bacterium of *Lactobacillus* were tested against the strain of G. *vaginalis* ATCC 10231 grown in BHI (*brain-heart infusion broth*) containing 2% (w/w) of gelatin, 0.5% of yeast extract, 0.1% of starch and 0.1% of glucose. Each strain was cultured in a culture MRS medium. All the dishes were incubated at 37°C over 48 hours under anaerobic conditions (*GasPak System*).
   The neutralized supernatants of each strain were added at different percentages to fresh BHI and inoculated with G. *vaginalis* ATCC 10231. The growth of the strain G. *vaginalis* alone and in the presence of different concentrations of neutralized supernatant from 0.5% to 4% was quantified by means of optical density at 600 nm (OD₆₀₀) after 24 hours and 48 hours of incubation at 37°C under microaerophilic conditions.
ii) *In vivo* said composition was tested in 35 patients divided in: Group A, 24 subjects and Group B (placebo) 11 subjects. The placebo consisted of vaginal tablets of equal weight containing exclusively microcrystalline cellulose and anhydrous calcium hydrogen phosphate (inert ingredients). The vaginal tablets of the composition and placebo were administered to the subjects of Group A and Group B (placebo) once per day for 7 consecutive nights before going to bed. Next, one tablet every 3 days per 3 weeks. The complete treatment lasted 4 weeks for both the groups.
This study was performed in order to at first assess the ability of the selected lactobacilli to antagonize in vitro *Gardnerella vaginalis* so as to be combined with a strain capable to inhibit *Escherichia coli,* thus exerting a possible potential use even in aerobic vaginitis (AV). The second step of the study was a human pilot test in women with BV by using a combination of the most promising and active bacteria.
Methods. For this purpose, neutralized supernatants of each lactobacillus were assessed at percentages ranging from 0.5% to 4% for their capability to hindering the growth of G. *vaginalis* ATCC 10231.
The bacterium capable to exert the greatest inhibition was next tested along with L. plantarum LP01 in a placebo-controlled pilot test, with human interference, which involved 34 female subjects (age between 18 and 50, mean 34.7 ± 8.9, no menopausal women) diagnosed with BV. The two microorganisms L. fermentum LF15 (DSM 26955) and L. plantarum LP01 (LMG P-21021) were administered in the vagina by means of slow-release vaginal tablets also comprising 50 mg of tara gum. The amount of each strain was 400 millions of viable cells per dose. The women were instructed to apply a vaginal tablet once per day for 7 consecutive nights, next a tablet every 3 nights for a further application of 3 weeks (acute stage) and, finally, a tablet per week in order to maintain a long-term vaginal colonization against possible relapses. A clinical examination was performed and the Nugent score quantified for each patient at the beginning (d0), after 28 days (d28) and at the end of the second month for preventing relapses (d56). A statistical comparison between d28, or d56, and d0, as well as between d56 and d28 was performed in order to quantify the efficacy against possible relapses.
Results. *L. fermentum* LF15 showed the maximum inhibitory activity in vitro against G. *vaginalis* ATCC 10231 after 24 and 48 hours.
In the human test, the two selected lactobacilli, namely *L*. *fermentum* LF15 and *L*. *plantarum* LP01, significantly reduced the Nugent score below the threshold of 7 after 28 days in 22 of 24 patients in the Group with active agent (91.7%, p < 0.001). Eight women (33.3%) recorded a Nugent score between 4 and 6, evidence of an intermediate situation, whereas the remaining fourteen (58.3%) showed an assessment less to 4, thus suggesting the restoration of a physiological vaginal microbiota. At the end of the second month, only 4 women recorded a Nugent score greater than 7 and which can be defined as BV (16.7%, p = 0.065 relative to d28). In the placebo no significant differences were recorded at any time.
The present study suggests the ability of the two strains *L*. *fermentum* LF15 and *L*. *plantarum* LP01 to effectively hinder acute infections by Gardnerella and to significantly ameliorate the relative annoying symptoms in a very high percentage of women.
This result can be also ascribed to the presence of tara gum, capable to form a mechanical barrier against Gardnerella over the surface of the vaginal mucosa as primary mechanism.
Additionally, a long-term physiological protection is established due to the supplementation of the two lactobacilli in the vaginal microbiota and to their adhesion to the mucosal epithelial cells.
In light of the additional in vitro inhibitory activity against *E. coli,* their use in aerobic vaginitis (AV) is very useful.
The present study shows the ability of *L*. *fermentum* LF15 (DSM 26955) and *L. plantarum* LP01 (LMG P-21021) to effectively hinder acute infections by Gardnerella and to significantly ameliorate the annoying symptoms reported by women with BV. This evidence is ascribed to the presence of tara gum, an ingredient capable to create a mechanical barrier against the adhesion of Gardnerella and other possible detrimental microbes to the surface of the vaginal mucosa. The overall adaptation and tolerability were very good, since only one exclusion in the placebo was recorded due to the deviation from the protocol and no adverse events occurred.
A long-term physiological protection due to the supplementation of the two lactobacilli in the vaginal microbiota and their adhesion to the mucosal epithelial cells is also shown, since a very low percentage of relapses was recorded in the group with active agent during the second month of the protocol. In this regards, the use of arabinogalactan and fructo-oligosaccharides (FOS), two fibers capable to improve the vaginal colonization by the two lactobacilli, has a role in the long-term barrier against reinfections recorded in the second month of the study protocol.
Although the group with placebo included a lesser number of subjects relative to the group with active agent, the placebo effect was very low since no statistically significant differences were recorded neither after 28 days (p = 0.619) nor at the end of the protocol (p = 0.823), thus suggesting that the improvement recorded in the subjects receiving the two lactobacilli is specific and attributable to the global effect of the tested composition.
It is also interesting to underline the fact that an in vitro function exerted by a selected *Lactobacillus,* such as the inhibition of the Gardnerella growth by means of specific action mechanisms, was advantageously confirmed during this pilot test in female subjects diagnosed with BV. This suggests that the in vitro antagonism against G. *vaginalis* can prove to be a criterion for effectively selecting lactobacilli with a protective action in the event of BV, as regards both an acute treatment and the long-term prevention of relapses. In the in vitro step of the study, the selection of a proper growth medium suitable for the Gardnerella inhibition test was another key point since presumably certain components existing in MRS, the medium commonly used for growing lactobacilli before the inhibition assessment, can be able to promote the Gardnerella growing. In fact, the growth of Gardnerella in BHI alone was relatively poor, particularly after 48 hours.

In light of the in vitro inhibitory activity against *E. coli* primarily exerted by *L*. *plantarum* LP01 (29), the potential use of its combination with *L*. *fermentum* LF15 in aerobic vaginitis (AV), can prove to be highly useful and interesting. In fact, AV is mainly related to Escherichia coli, but also *Streptococcus agalactiae* and *Staphylococcus aureus* are involved. The standard AV treatment consists of oral or vaginal antibiotics, although it is unable to automatically restore a normal flora characterized by a high concentration of lactobacilli, thus easing relapses and reinfections. In conclusion, the study also represents an effective approach for preventing possible relapses caused by *Gardnerella vaginalis* (bacterial vaginosis) or *Escherichia coli* (aerobic vaginitis).

## Claims

1. A composition comprising a mixture of bacteria, which comprises or, alternatively, consists of:
- at least a strain of bacterium selected from the group comprising the strain *Lactobacillus plantarum* LMG P-21021 (LP01) and the strain *Lactobacillus plantarum* LMG P-21020 (LP02), combined with:
- at least a strain of bacterium selected from the group comprising the strain of bacteria *Lactobacillus fermentum* DSM 26955 (LF15) and the strain of bacteria *Lactobacillus fermentum* DSM 26956 (LF16),
for use in the preventive and/or curative treatment of bacterial vaginosis, vaginitis and vaginal infections related with coliform pathogens and/or Gardnerella.

2. The composition for use according to claim 1, for the treatment of recurrent or relapsing vaginosis.

3. The composition for use according to claims 1 or 2, wherein said mixture of bacteria comprises or, alternatively, consists of the strain of bacterium *Lactobacillus plantarum* LMG P-21021 (LP01), combined with at least a strain of bacterium selected from the group comprising the strain of bacterium *Lactobacillus fermentum* DSM 26955 (LF15) and the strain of bacterium *Lactobacillus fermentum* DSM 26956 (LF16).

4. The composition for use according to claim 3, wherein said mixture of bacteria comprises or, alternatively, consists of the strain of bacterium *Lactobacillus plantarum* LMG P-21021 (LP01) and the strain of bacterium *Lactobacillus fermentum* DSM 26955 (LF15).

5. The composition for use according to claims 1 or 2, wherein said mixture of bacteria comprises or, alternatively, consists of the strain of bacterium *Lactobacillus plantarum* LMG P-21020 (LP02), combined with at least a strain of bacterium selected from the group comprising the strain of bacterium *Lactobacillus fermentum* DSM 26955 (LF15) and the strain of bacterium *Lactobacillus fermentum* DSM 26956 (LF16).

6. The composition for use according to claim 5, wherein said mixture of bacteria comprises or, alternatively, consists of the strain of bacteria *Lactobacillus plantarum* LMG P-21020 (LP02) and the strain of bacteria *Lactobacillus fermentum* DSM 26956 (LF16).

7. The composition for use according to any one of the preceding claims, wherein said composition further comprises at least a gelling substance, preferably having a marked muco-adhesive property, such as a natural gum or a plant gum and/or a plant gelatin selected from the group comprising a tannate or a gelatin tannate, an alginate, a xyloglucan or xylogel, a guar gum, a tara gum, an acacia, carob, oat, bamboo fiber, citrus fruit fibers and glucomannans. Preferably said gelling substance is selected from natural galactomannans; preferably, the gelling substance is selected from guar gums.

8. The composition for use according to any one of the preceding claims, wherein said composition further comprises at least a fiber with prebiotic activity selected from the group comprising inulin, fructo-oligosaccharides (FOS), galacto and trans-galacto-oligosaccharides (GOS and TOS), gluco-oligosaccharides (GOSα), xylo-oligosaccharides, (XOS), chitosan-oligosaccharides (COS), soy-oligosaccharides (SOS), isomalto-oligosaccharides (IMOS), resistant starch, pectins, psyllium, arabino-galactans, gluco-mannans and galacto-mannans; preferably said fiber with prebiotic activity is selected from fructo-oligosaccharides (FOS), arabinogalactans or mixtures thereof.

9. The composition for use according to any of the preceding claims, wherein said composition comprises a mixture comprising or, alternatively, consisting of:
- a strain of bacterium *Lactobacillus plantarum* LMG P-21021 (LP01), and
- a strain of bacterium *Lactobacillus fermentum* DSM 26955 (LF15); said composition further comprises:
- a tara gum, and
- an arabinogalactan fiber, and
- a fructo-oligosaccharide fiber.

10. The composition for use according to claim 9, wherein the strain of bacterium *Lactobacillus plantarum* LMG P-21021 (LP01) and the strain of bacterium *Lactobacillus fermentum* DSM 26955 (LF15) are each present in a concentration comprised from 1x10⁷ to 1x10¹⁰ CFU/g of composition, preferably from 1x10⁸ to 1x10⁹ CFU/g of composition.

11. The composition for use according to claims 9 or 10, wherein said two strains of bacterium are present in the composition in a weight ratio comprised from 1:3 to 3:1, preferably from 1:2 to 2:1, for example 1:1.

12. The composition for use according to any of claims 9-11, wherein the tara gum is present in an amount comprised from 1 to 10% by weight, relative to the weight of the composition, preferably from 3 to 8%, for example 5%.

13. The composition for use according to any of claims 9-12, wherein said arabinogalactan fiber is present in an amount comprised from 10 to 45% by weight, relative to the weight of the composition, preferably from 25 to 40%, for example 35%; and said fructo-oligosaccharide fiber is present in an amount comprised from 10 to 40% by weight, relative to the weight of the composition, preferably from 15 to 35%, for example 25%.

14. The composition for use according to any of claims 9-13, wherein the weight ratio between arabinogalactan fiber and fructo-oligosaccharide fiber is comprised from 1:2 to 2:1, preferably from 1:1.5 to 1.5:1.

15. A strain of bacterium belonging to the species *Lactobacillus fermentum* deposited by Probiotical S.p.A. Company on 03.01.2013 at the DSMZ center and having deposit number DSM 26955 (LF15) and a strain of bacterium belonging to the species *Lactobacillus fermentum* deposited by Probiotical S.p.A. Company on 03.01.2013 at the DSMZ center and having deposit number DSM 26956 (LF16), for use in the preventive and/or curative treatment of bacterial vaginosis, vaginitis and vaginal infections related with pathogenic coliform bacteria and/or Gardnerella, preferably in the treatment of recurrent or relapsing vaginosis.

## Patentansprüche

1. Zusammensetzung umfassend eine Bakterienmischung, die umfasst bzw. besteht aus:
- mindestens einem Bakterienstamm ausgewählt aus der Gruppe, die den Bakterienstamm *Lactobacillus plantarum* LMG P-21021 (LP01) und den Bakterienstamm *Lactobacillus plantarum* LMG P-21020 (LP02) umfasst, in Kombination mit:
- mindestens einem Bakterienstamm ausgewählt aus der Gruppe, die den Bakterienstamm *Lactobacillus fermentum* DSM 26955 (LF15) und den Bakterienstamm *Lactobacillus fermentum* DSM 26956 (LF16) umfasst,
zur Verwendung bei vorbeugenden und/oder heilenden Behandlungen von bakterieller Vaginose, Vaginitis und Vaginalinfektionen, die in Zusammenhang mit coliformen Pathogenen und/oder Gardnerella stehen.

2. Zusammensetzung zur Verwendung nach Anspruch 1, zur Behandlung von wiederkehrender Vaginose oder zur Behandlung von Vaginose-Rückfällen.

3. Zusammensetzung zur Verwendung nach Anspruch 1 oder 2, wobei die Bakterienmischung den Bakterienstamm *Lactobacillus plantarum* LMG P-21021 (LP01) umfasst bzw. daraus besteht, in Kombination mit mindestens einem Bakterienstamm ausgewählt aus der Gruppe, die den Bakterienstamm *Lactobacillus fermentum* DSM 26955 (LF15) und den Bakterienstamm *Lactobacillus fermentum* DSM 26956 (LF16) umfasst.

4. Zusammensetzung zur Verwendung nach Anspruch 3, wobei die Bakterienmischung den Bakterienstamm *Lactobacillus plantarum* LMG P-21021 (LP01) und den Bakterienstamm *Lactobacillus fermentum* DSM 26955 (LF15) umfasst bzw. daraus besteht.

5. Zusammensetzung zur Verwendung nach Anspruch 1 oder 2, wobei die Bakterienmischung den Bakterienstamm *Lactobacillus plantarum* LMG P-21020 (LP02) umfasst bzw. daraus besteht, in Kombination mit mindestens einem Bakterienstamm ausgewählt aus der Gruppe, die den Bakterienstamm *Lactobacillus fermentum* DSM 26955 (LF15) und den Bakterienstamm *Lactobacillus fermentum* DSM 26956 (LF16) umfasst.

6. Zusammensetzung zur Verwendung nach Anspruch 5, wobei die Bakterienmischung den Bakterienstamm *Lactobacillus plantarum* LMG P-21020 (LP02) und den Bakterienstamm *Lactobacillus fermentum* DSM 26956 (LF16) umfasst bzw. daraus besteht.

7. Zusammensetzung zur Verwendung nach einem der vorhergehenden Ansprüche, wobei die Zusammensetzung ferner mindestens eine gelierende Substanz, vorzugsweise mit ausgeprägter mukoadhäsiver Eigenschaft, umfasst, wie einen natürlichen Gummi oder Pflanzengummi und/oder pflanzliche Geliermittel ausgewählt aus der Gruppe umfassend Tannat oder Gelatinetannat, Alginat, Xyloglucan oder Xylogel, Guargummi, Taragummi, Akazienfasern, Johannisbrotfasern, Haferfasern, Bambusfasern, Fasern von Zitrusfrüchten und Glucomannane. Die gelierende Substanz wird vorzugsweise unter den natürlichen Galactomannanen ausgewählt; die gelierende Substanz wird vorzugsweise aus Guargummen ausgewählt.

8. Zusammensetzung zur Verwendung nach einem der vorhergehenden Ansprüche, wobei die Zusammensetzung ferner mindestens eine Faser mit präbiotischer Aktivität umfasst, ausgewählt aus der Gruppe umfassend Inulin, Fructooligosaccharide (FOS), Galacto- und Transgalactooligosaccharide (GOS und TOS), Glucooligosaccharide (GOSα), Xylooligosaccharide (XOS), Chitosanoligosaccharide (COS), Soja-Oligosaccharide (SOS), Isomaltooligosaccharide (IMOS), resistente Stärke, Pektine, Psyllium, Arabinogalactane, Glucomannane und Galactomannane; die Faser mit präbiotischer Aktivität wird vorzugsweise unter den Fructooligosacchariden (FOS), Arabinogalactanen oder Mischungen hiervon ausgewählt.

9. Zusammensetzung zur Verwendung nach einem der vorhergehenden Ansprüche, wobei die Zusammensetzung eine Mischung umfasst, die umfasst bzw. besteht aus:
- einem Bakterienstamm *Lactobacillus plantarum* LMG P-21021 (LP01) und
- einem Bakterienstamm *Lactobacillus fermentum* DSM 26955 (LF15); wobei die Zusammensetzung ferner umfasst:
- Taragummi und
- Arabinogalactanfasern und
- Fructooligosaccharidfasern.

10. Zusammensetzung zur Verwendung nach Anspruch 9, wobei der Bakterienstamm *Lactobacillus plantarum* LMG P-21021 (LP01) und der Bakterienstamm *Lactobacillus fermentum* DSM 26955 (LF15) jeweils in einer Konzentration von 1x10⁷ bis 1x10¹⁰ KBE/g Zusammensetzung, vorzugsweise von 1x10⁸ bis 1x10⁹ KBE/g Zusammensetzung, vorliegen.

11. Zusammensetzung zur Verwendung nach Anspruch 9 oder 10, wobei die beiden Bakterienstämme in einer Zusammensetzung in einem Gewichtsverhältnis von 1:3 bis 3:1, vorzugsweise von 1:2 bis 2:1, zum Beispiel 1:1, vorliegen.

12. Zusammensetzung zur Verwendung nach einem der Ansprüche 9 bis 11, wobei Taragummi bezogen auf das Gewicht der Zusammensetzung in einer Menge von 1 bis 10 Gewichtsprozent, vorzugsweise 3 bis 8 Gewichtsprozent, zum Beispiel 5 Gewichtsprozent, vorliegt.

13. Zusammensetzung zur Verwendung nach einem der Ansprüche 9 bis 12, wobei die Arabinogalactanfasern bezogen auf das Gewicht der Zusammensetzung in einer Menge von 10 bis 45 Gewichtsprozent, vorzugsweise 25 bis 40 Gewichtsprozent, zum Beispiel 35 Gewichtsprozent, vorliegen; und die Fructooligosaccharidfasern bezogen auf das Gewicht der Zusammensetzung in einer Menge von 10 bis 40 Gewichtsprozent, vorzugsweise 15 bis 35 Gewichtsprozent, zum Beispiel 25 Gewichtsprozent, vorliegen.

14. Zusammensetzung zur Verwendung nach einem der Ansprüche 9 bis 13, wobei das Gewichtsverhältnis zwischen Arabinogalactanfasern und Fructooligosaccharidfasern 1:2 bis 2:1, vorzugsweise 1:1,5 bis 1,5:1, beträgt.

15. Bakterienstamm, der zur Species *Lactobacillus fermentum* gehört, vom Unternehmen Probiotical S.p.A. bei der DSMZ am 3. Januar 2013 hinterlegt wurde und die Hinterlegungsnummer DSM 26955 (LF15) aufweist, und ein Bakterienstamm, der zur Species *Lactobacillus fermentum* gehört, vom Unternehmen Probiotical S.p.A. bei der DSMZ am 3. Januar 2013 hinterlegt wurde und die Hinterlegungsnummer DSM 26956 (LF16) aufweist, zur Verwendung bei vorbeugenden und/oder heilenden Behandlungen von bakterieller Vaginose, Vaginitis und Vaginalinfektionen, die in Zusammenhang mit pathogenen coliformen Bakterien und/oder Gardnerella stehen, vorzugweise zur Verwendung bei der Behandlung von wiederkehrender Vaginose oder bei der Behandlung von Vaginose-Rückfällen.

## Revendications

1. Composition comprenant un mélange de bactéries, qui comprend ou, en variante, consiste en :
- au moins une souche bactérienne choisie dans le groupe comprenant la souche *Lactobacillus plantarum* LMG P-21021 (LP01) et la souche *Lactobacillus plantarum* LMG P-21020 (LP02), combinée avec :
- au moins une souche bactérienne choisie dans le groupe comprenant la souche bactérienne *Lactobacillus fermentum* DSM 26955 (LF15) et la souche bactérienne *Lactobacillus fermentum* DSM 26956 (LF16),
pour une utilisation dans le traitement préventif et/ou curatif d'une vaginose bactérienne, d'une vaginite et d'infections vaginales liées à des pathogènes coliformes et/ou à Gardnerella.

2. Composition pour une utilisation selon la revendication 1, pour le traitement d'une vaginose récurrente ou récidivante.

3. Composition pour une utilisation selon la revendication 1 ou 2, dans laquelle ledit mélange de bactéries comprend ou, en variante, consiste en la souche bactérienne *Lactobacillus plantarum* LMG P-21021 (LP01), combinée avec au moins une souche bactérienne choisie dans le groupe comprenant la souche bactérienne *Lactobacillus fermentum* DSM 26955 (LF15) et la souche bactérienne *Lactobacillus fermentum* DSM 26956 (LF16) .

4. Composition pour une utilisation selon la revendication 3, dans laquelle ledit mélange de bactéries comprend ou, en variante, consiste en la souche bactérienne *Lactobacillus plantarum* LMG P-21021 (LP01) et la souche bactérienne *Lactobacillus fermentum* DSM 26955 (LF15).

5. Composition pour une utilisation selon la revendication 1 ou 2, dans laquelle ledit mélange de bactéries comprend ou, en variante, consiste en la souche bactérienne *Lactobacillus plantarum* LMG P-21020 (LP02), combinée avec au moins une souche bactérienne choisie dans le groupe comprenant la souche bactérienne *Lactobacillus fermentum* DSM 26955 (LF15) et la souche bactérienne *Lactobacillus fermentum* DSM 26956 (LF16) .

6. Composition pour une utilisation selon la revendication 5, dans laquelle ledit mélange de bactéries comprend ou, en variante, consiste en la souche bactérienne *Lactobacillus plantarum* LMG P-21020 (LP02) et la souche bactérienne *Lactobacillus fermentum* DSM 26956 (LF16).

7. Composition pour une utilisation selon l'une quelconque des revendications précédentes, laquelle composition comprend en outre au moins une substance gélifiante, ayant de préférence une propriété marquée d'adhérence aux muqueuses, telle que une gomme naturelle ou une gomme végétale et/ou une gélatine végétale choisie dans le groupe comprenant un tannate ou un tannate de gélatine, un alginate, un xyloglucane ou xylogel, une gomme de guar, une gomme de tara, une fibre d'acacia, de caroube, d'avoine, de bambou, des fibres d'agrumes et des glucomannanes, de préférence ladite substance gélifiante est choisie parmi les galactomannanes naturels ; de préférence la substance gélifiante est choisie parmi les gommes de guar.

8. Composition pour une utilisation selon l'une quelconque des revendications précédentes, laquelle composition comprend en outre au moins une fibre ayant une activité prébiotique choisie dans le groupe comprenant l'inuline, les fructo-oligosaccharides (FOS), les galacto- et trans-galacto-oligosaccharides (GOS et TOS), les gluco-oligosaccharides (GOSα), les xylo-oligosaccharides (XOS), les chitosane-oligosaccharides (COS), les soja-oligosaccharides (SOS), les isomalto-oligosaccharides (IMOS), l'amidon résistant, les pectines, le psyllium, les arabino-galactanes, les gluco-mannanes et les galacto-mannanes ; de préférence ladite fibre ayant une activité prébiotique est choisie parmi les fructo-oligosaccharides (FOS), les arabinogalactanes et leurs mélanges.

9. Composition pour une utilisation selon l'une quelconque des revendications précédentes, laquelle composition comprend un mélange comprenant ou, en variante, consistant en :
- une souche bactérienne de *Lactobacillus plantarum* LMG P-21021 (LP01), et
- une souche bactérienne de *Lactobacillus fermentum* DSM 26955 (LF15) ;
laquelle composition comprend en outre :
- une gomme de tara, et
- une fibre d'arabino-galactane, et
- une fibre de fructo-oligosaccharide.

10. Composition pour une utilisation selon la revendication 9, dans laquelle la souche bactérienne de *Lactobacillus plantarum* LMG P-21021 (LP01) et la souche bactérienne de *Lactobacillus fermentum* DSM 26955 (LF15) sont présentes chacune à une concentration comprise de 1 x 10⁷ à 1 x 10¹⁰ UFC/g de composition, de préférence de 1 x 10⁸ à 1 x 10⁹ UFC/g de composition.

11. Composition pour une utilisation selon la revendication 9 ou 10, dans laquelle lesdites deux souches bactériennes sont présentes dans la composition en un rapport en poids compris de 1/3 à 3/1, de préférence de 1/2 à 2/1, par exemple de 1/1.

12. Composition pour une utilisation selon l'une quelconque des revendications 9 à 11, dans laquelle la gomme de tara est présente en une quantité comprise de 1 à 10 % en poids, par rapport au poids de la composition, de préférence comprise de 3 à 8 %, par exemple de 5 %.

13. Composition pour une utilisation selon l'une quelconque des revendications 9 à 12, dans laquelle ladite fibre d'arabino-galactane est présente en une quantité comprise de 10 à 45 % en poids, par rapport au poids de la composition, de préférence de 25 à 40 %, par exemple de 35 % ; et ladite fibre de fructo-oligosaccharide est présente en une quantité comprise de 10 à 40 % en poids, par rapport au poids de la composition, de préférence de 15 à 35 %, par exemple de 25 %.

14. Composition pour une utilisation selon l'une quelconque des revendications 9 à 13, dans laquelle le rapport en poids entre la fibre d'arabino-galactane et la fibre de fructo-oligosaccharide est compris de 1/2 à 2/1, de préférence de 1/1,5 à 1,5/1.

15. Souche bactérienne appartenant à l'espèce *Lactobacillus fermentum,* déposée par la société Probiotical S.p.A. le 03.01.2013 au centre DSMZ et ayant le numéro de dépôt DSM 26955 (LF15), et souche bactérienne appartenant à l'espèce *Lactobacillus fermentum,* déposée par la société Probiotical S.p.A. le 03.01.2013 au centre DSMZ et ayant le numéro de dépôt DSM 26956 (LF16), pour une utilisation dans le traitement préventif et/ou curatif d'une vaginose bactérienne, d'une vaginite et d'infections vaginales liées à des bactéries coliformes pathogènes et/ou à Gardnerella, de préférence dans le traitement d'une vaginose récurrente ou récidivante.
